(19) Europäisches Patentamt
European Patent Office
Office européen des brevets

(11) Publication number: 0 180 252 B1

(12) EUROPEAN PATENT SPECIFICATION

(45) Date of publication of patent specification: 26.04.89

(51) Int. Cl.$^4$: C 07 D 205/08

(21) Application number: 85200793.9

(22) Date of filing: 15.07.82

(60) Publication number of the earlier application in accordance with Art. 76 EPC: 0 070 204

# (54) Process of preparing azetidinone compounds.

(30) Priority: 15.07.81 JP 111108/81
01.12.81 JP 193947/81
03.12.81 JP 195470/81
04.12.81 JP 196037/81
28.12.81 JP 211416/81

(43) Date of publication of application:
07.05.86 Bulletin 86/19

(45) Publication of the grant of the patent:
26.04.89 Bulletin 89/17

(84) Designated Contracting States:
AT BE CH DE FR GB IT LI NL SE

(56) References cited:
EP-A-0 003 960
EP-A-0 078 026
TETRAHEDRON LETTERS, vol. 23, no. 22, 1982, pages 2293-2296, Pergamon Press, Oxford, GB; P.J. REIDER et al.: "Total synthesis of thienamycin: A new approach from aspartic acid"
Houben-Weil: Methoden der Organischen Chemie, G. Thieme, Stuttgart, BD. VI/1a,7; S. 912-914; BD. XIII/2b, S. 130-132

(73) Proprietor: SUMITOMO PHARMACEUTICALS COMPANY, LIMITED
40, Dosho-machi 2-chome
Higashi-ku Osaka-shi Osaka-fu (JP)

(72) Inventor: Sunagawa, Makoto
No. 11-8-106, Sonehigashi-machi 2-chome
Toyonaka-shi Osaka (JP)
Inventor: Matsumura, Haruki
7-96 Aoyama
Nara-shi Nara (JP)
Inventor: Inoue, Takaaki
No. 14-7, Mefe 2-chome
Takarazuka-shi Hyogo (JP)
Inventor: Enomoto, Masao
No. 14-7, Mefe 2-chome
Takarazuka-shi Hyogo (JP)

(74) Representative: Diamond, Bryan Clive et al
Gee & Co. Chancery House Chancery Lane
London WC2A 1QU (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 180 252 B1

**Description**

This invention relates to the preparation of certain substituted azetidinone compounds.

The compounds thus prepared are useful as starting materials for the process of preparing carboxylic β-lactam compounds described and claimed in our Specification EP 0070204A.

The compounds prepared by the process of the invention include those of the following general formula:

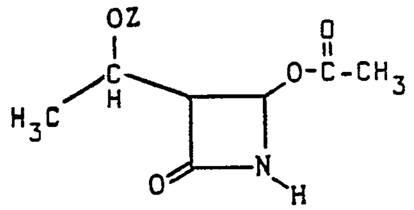

(a)

wherein Z is a conventional protecting group for a hydroxyl group as described in our aforesaid specification, and including, for example, a $C_1$—$C_4$ alkoxy carbonyl group such as *tert*-butyloxycarbonyl, a $C_1$—$C_4$ halogenoalkoxycarbonyl group such as 2-iodoethyloxycarbonyl or 2,2,2-trichlorethyloxycarbonyl, an aralkyloxycarbonyl group such as benzyloxycarbonyl, *p*-methoxybenzyloxycarbonyl, *o*-nitrobenzyloxycarbonyl or *p*-nitrobenzyloxycarbonyl, a tri($C_1$—$C_4$)alkylsilyl group such as *tert*-butyldimethylsilyl or trimethylsilyl, a $C_4$—$C_{10}$ *tert*-alkyl group such as *tert*-butyl and a substituted or unsubstituted mono-, di or tri-phenylmethyl group such as benzyl, *p*-methoxybenzyl, diphenylmethyl, di(*p*-anisyl)methyl or trityl.

According to a broad aspect of the present invention, these compounds and derivatives thereof are prepared from an azetidinone substituted in the 1, 3 and 4 positions, by a process as follows:

A process for preparing a compound of the formula:

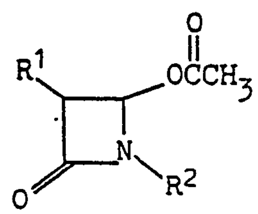

(a')

wherein $R^1$ is 1-hydroxyethyl, a protected 1-hydroxyethyl wherein the hydroxy group is protected by a conventional protecting group for a hydroxy group, or vinyl, and $R^2$ is hydrogen, a mono- or diarylmethyl, comprises oxidatively decarboxylating a carboxylic acid compound of the formula:

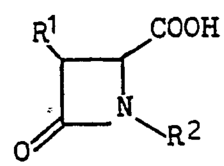

(b)

wherein $R^1$ and $R^2$ are as defined above, with lead tetraacetate in an inert solvent in the presence or absence of a base or a salt.

The starting material of general formula (b), and some derivatives thereof, namely for the case where $R^1$ is hydroxyethyl (the hydroxyethyl group may also be a protected group, or may be a vinyl group) can be prepared as follows; a process for preparing an azetidinone compound of the formula:

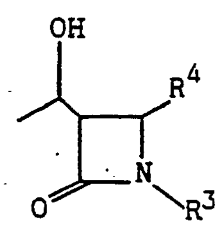

(b')

wherein $R^3$ is mono- or diarylmethyl and $R^4$ is acetoxy, carboxyl or a protected carboxyl group, comprises subjecting a compound of the formula:

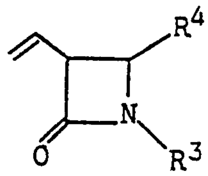

(c)

wherein $R^3$ and $R^4$ are as defined above, to oximercuration with mercuric acetate and then to reductive demercuration with a reducing agent in an inert solvent.

The complete process of preparing a compound of the aforesaid general formula (a) can be carried out preferably by one of the three following processes (i) to (iii), in all of which $R^3$ again represents mono- or diarylmethyl:

A process for preparing a compound of the general formula:

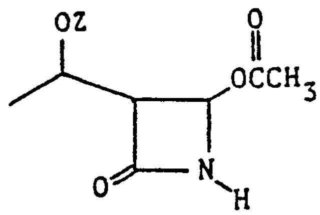

(a)

wherein Z is a conventional protecting group for a hydroxyl group, comprises:

i) oxidizing a carboxylic acid compound of the formula:

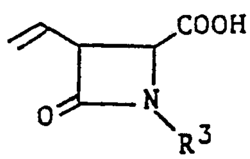

(d)

with lead tetraacetate in an inert solvent to produce a compound of the formula:

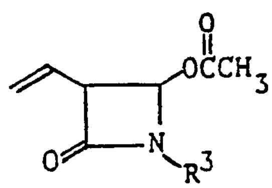

(e)

and then treating the compound of the formula (e) thus obtained with mercuric acetate, reducing the oxidized compound with a reducing agent in an inert solvent to produce a compound of the formula:

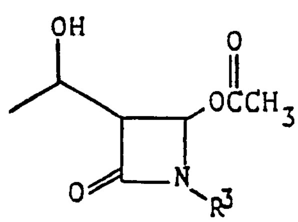

(f)

protecting the hydroxy group of the compound of the formula (f) thus obtained by a conventional hydroxy-protecting agent, and oxidizing the protected compound with ceric ammonium nitrate in an inert solvent; or

(ii) treating a carboxylic acid compound of the formula (d) above with mercuric acetate and then reducing the oxidized compound with a reducing agent in an inert solvent to produce a compound of the formula:

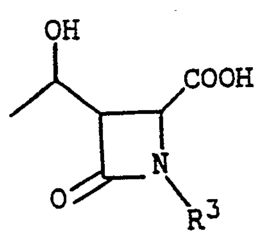

(g)

oxidizing the compound of the formula (g) thus obtained with lead tetraacetate in an inert solvent to produce a compound of the formula:

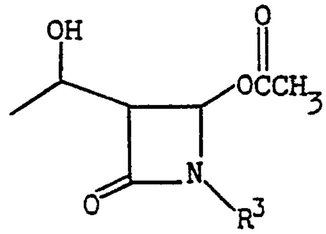

(h)

protecting the hydroxy group of the compound of the formula (h) thus obtained by a conventional hydroxy-protecting agent, and oxidizing the protected compound by ceric ammonium nitrate in an inert solvent; or (iii) treating a compound of the formula:

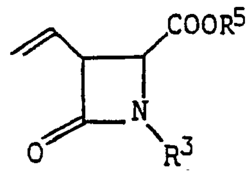

(d')

wherein $R^5$ is $C_1$—$C_4$ alkyl
or aryl-($C_1$—$C_4$) alkyl, with mercuric acetate and then reducing the oxidized compound with a reducing agent in an inert solvent, protecting the hydroxy group of the resulting compound by a conventional hydroxy-protecting agent, removing the protecting group for carboxyl group ($R^5$) from the resulting hydroxy-protected compound to produce a compound of the formula:

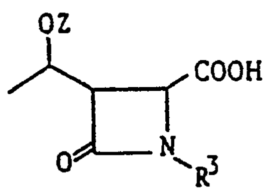

(j)

wherein Z is as defined above, oxidizing the compound of the formula (j) thus obtained with ceric ammonium nitrate in an inert solvent to produce a compound of the formula:

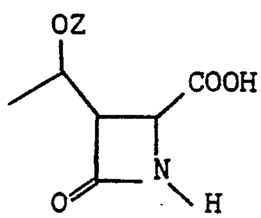

(k)

wherein Z is as defined above, and oxidizing the compound of the formula (k) thus obtained with lead tetraacetate.

These methods are further described as follows:

Process (i):

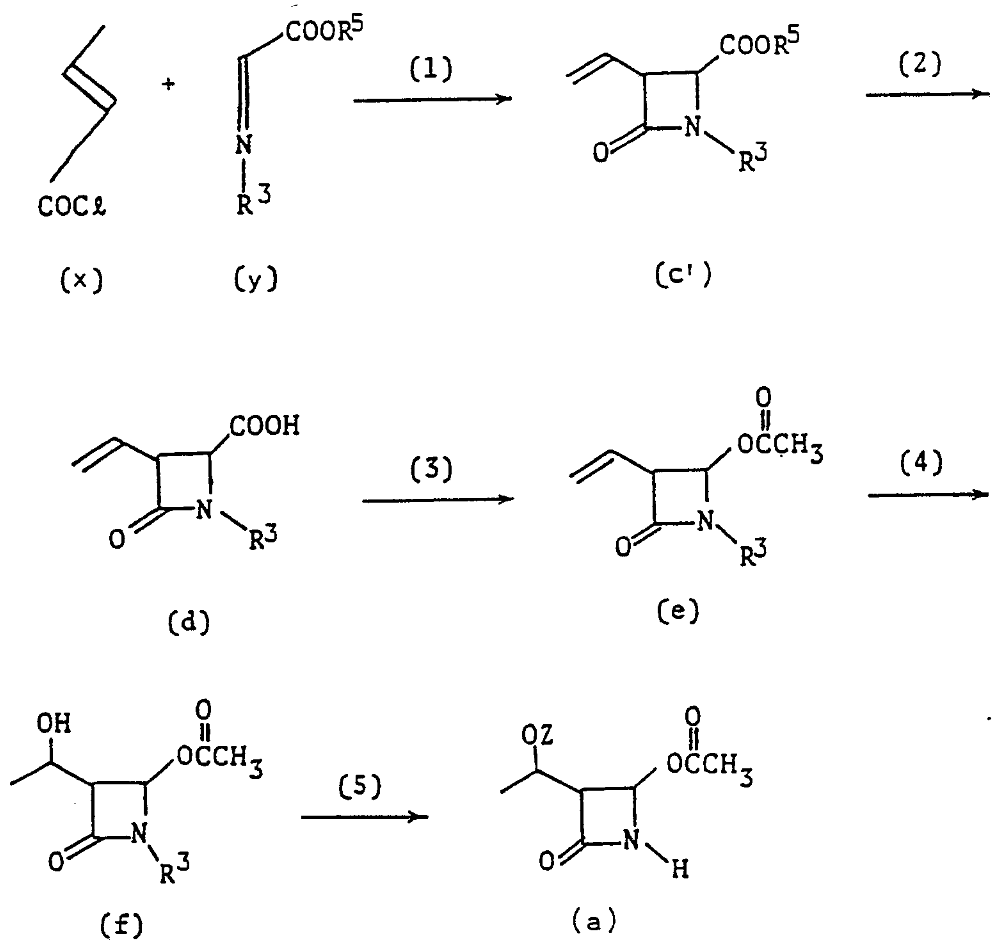


(x) (y) (c')

(d) (e)

(f) (a)

wherein $R^5$ is $C_1$—$C_4$ alkyl or aryl-($C_1$—$C_4$)alkyl, $R^3$ is substituted or unsubstituted mono- or diphenylmethyl, such as *p*-methoxybenzyl, di(*p*-anisyl)methyl or diphenylmethyl and Z is a conventional protecting group.

The conversion in each of the steps (1) to (5) can be achieved in the following manner.

(1) The β-lactam compound (c') can be prepared by reacting crotonoyl chloride with a Schiff base (which can be prepared from an ester derivative of glyoxylic acid and a corresponding primary amine in a conventional manner) in the presence of a base such as triethylamine, pyridine, lutidine or DBU, in an inert solvent such as a halogenated hydrocarbon (e.g., chloroform or methylene dichloride), aromatic hydrocarbon (e.g., benzene or toluene), ether (e.g., diethyl ether, dioxane or tetrahydrofuran) or a mixture thereof. The temperature for the addition-cyclization reaction can be varied from 0°C to 100°C.

(2) The hydrolysis of the ester compound (c') to the corresponding acid (d) can be achieved in a manner as conventionally employed for hydrolysis of an ester to the corresponding carboxylic acid. The hydrolysis can be easily achieved using an alkali or an acid.

(3) The carboxylic acid (d) can be converted into the acetoxy compound (e) by oxidative decarboxylation with lead tetraacetate. The oxidative decarboxylation can be performed by treating the compound (d) with lead tetraacetate in an amount of not less than 1 mole, preferably 1 to 3 moles, per mole of the carboxylic acid (d) in an inert solvent in the presence or absence of a base, such as pyridine or lutidine, or a salt, such as sodium acetate or potassium acetate.

Examples of suitable inert solvents are aromatic hydrocarbons such as benzene or toluene, acetic acid, dimethylformamide, hexamethyl phosphoric triamide, diethyl ether, tetrahydrofuran, dioxane or a mixture thereof.

The reaction temperature can be from 0°C to 100°C.

(4) The hydroxyethyl compound (f) can be obtained by oxidizing the vinyl group of the acetoxy compound (e) with mercuric acetate and then reducing the oxidized derivative with reducing agent such as sodium borohydride in an inert solvent such as water, tetrahydrofuran, dioxane, diethyl ether, acetonitrile

or a mixture thereof. The mercuric oxidation can be preferably achieved by reacting the acetoxy compound (e) with mercuric acetate in an amount of 1 to 2 moles per mole of the acetoxy compound at 0 to 100°C. The subsequent reductive demercuration can be carried out by reducing the resulting compound with sodium borohydride (0.25 to 5 molar amount) in the presence of an alkali metal hydroxide (preferably 0.5 to 5 molar amount) such as sodium hydroxide. The optimum temperature for the reduction reaction can be from −10°C to 40°C.

(5) The hydroxyethyl compound (f), for example, can be easily converted into a hydroxy-protected derivative thereof by using an acylating agent (e.g. *p*-nitrobenzyl chloroformate) in the present of a base. The azetidinone compound (a) can be prepared by oxidation of the hydroxy-protected derivative described above with ceric ammonium nitrate in an amount of 2 to 4 moles per mole of the hydroxy-protected derivative in an inert solvent such as dimethylformamide, acetonitrile, tetrahydrofuran, dioxane, ethanol, water or a mixture thereof at 0 to 60°C.

The azetidinone derivative (a) can also be prepared by the method shown in the following scheme of process (ii):

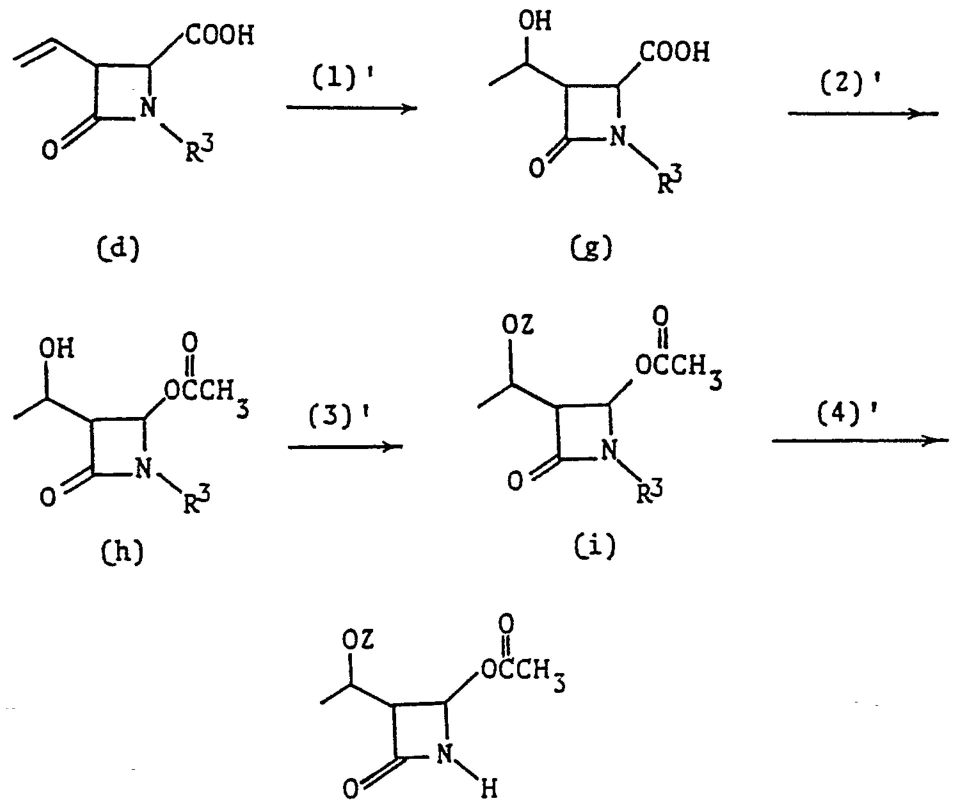


(d) (g)

(h) (i)

(a)

wherein $R^3$ and Z are as defined above.

The hydroxy-introducing reaction by oxymercuration (1)', the conversion reaction of carboxyl group to acetoxy group (2)', the protecting reaction of the hydroxy group (3)' and the elimination reaction of $R^3$ (4)' can be accomplished by the methods similar to those described above.

The azetidinone (a) can also be prepared according to the following scheme:

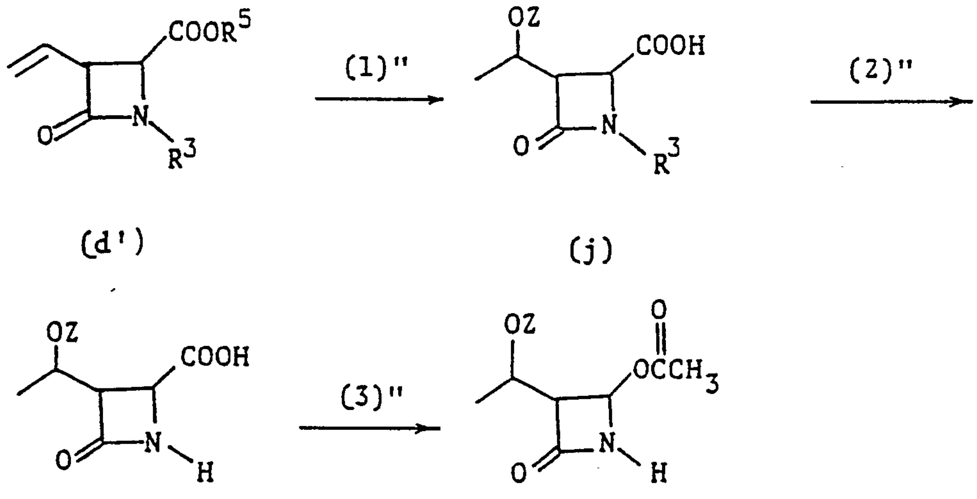


(d')

(j)

(k)

(a)

wherein $R^5$, $R^3$ and Z are as defined above.

The hydroxy-introducing reaction by oxymercuration, the hydroxy-protecting reaction and the elimination of a carboxyl-protecting group (1)'', the oxidative elimination of N-substituent (2)'' and the oxidative decarboxylation (3)'' can be accomplished by methods similar to those described above.

Each of the processes for preparing the β-lactam compounds of the formula (a) described above is a novel process. The β-lactam compounds of the formula (a) may exist in *threo* and *erthyro* forms with respect to the steric configuration of the hydroxy group in the protected hydroxyethyl group at the 3-position. These processes are characterized in that they are capable of producing the *threo* form with high selectivity.

Symbols used in the formulae below have the following meanings

DAM = 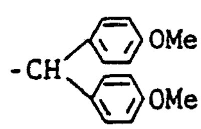

PMB = 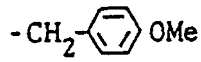

PNZ = 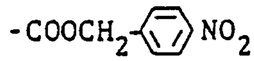

n-Bu = $-CH_2CH_2CH_2CH_3$

Ac = 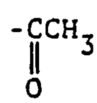

The following examples are given to illustrate the present invention. Percentages are by weight.

Example 1

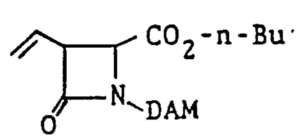

Di-*p*-anisylmethylamine (10 mg) and *n*-butylglyoxylate (7.3 g) in toluene were azeotropically dehydrated to give a Schiff base. To the toluene solution (600 ml), crotonyl chloride (5.1 g) in toluene (25 ml) was added dropwise at 70°C over 1 hour in the presence of triethylamine (6.2 g), followed by stirring for 2 hours. The reaction mixture was cooled, washed successively with water, 2N hydrochloric acid, aqueous sodium bicarbonate and again water, dried over anhydrous sodium sulfate and evaporated to give an oily residue which was then purified by silica gel chromatography to give 1-(di-*p*-anislymethyl)-3-ethenyl-4-*n*-butoxycarbonyl-2-azetidinone

$IR_{max}^{CHCl_3}$ ($cm^{-1}$): 1758, 1615, 1180, 1030, 930, 825
NMRδ ($CDCl_3$): 0.87(3H, br, t, J=6), 1.0-1.7(4H, m), 3.78(6H, s), 5.1-5.8(3H, m), 5.77(1H, s)

Example 2

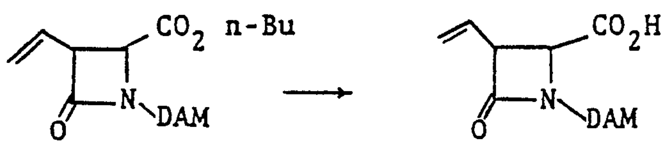

A solution of 1-(di-*p*-anisylmethyl)-3-ethyl-4-*n*-butoxycarbonyl-2-azetidinone (0.5 g) in 1N sodium hydroxide (1.2 ml), tetrahydrofuran (15 ml) and methanol (15 ml) was stirred for 2 hours at room temperature. After addition of 2N hydrochloric acid (0.7 ml), the reaction mixture was concentrated to a quarter of its original volume, diluted with water and extracted with diethyl ether. The ether extract was reextracted with an aqueous alkaline solution. The aqueous alkaline layer was acidified with hydrochloric acid and extracted with diethyl ether. The ether extract was washed with water, dried over sodium sulfate and evaporated to give 1-(di-*p*-anisylmethyl)-3-ethenyl-4-carboxyl-2-azetidinone.

$IR_{max}^{CHCl_3}$ ($cm^{-1}$): 1753, 1612, 1297, 1245, 1170, 1109, 1027, 828
NMRδ ($CDCl_3$): 3.80(6H, s), 5.1-5.9(3H, m), 5.83(1H, s), 8.64(1H, s)

Example 3

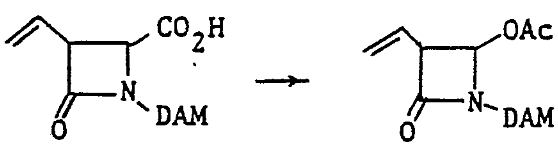

To a solution of 1-(di-*p*-anisylmethyl)-3-ethenyl-4-carboxy-2-azetidinone (1.5 g) and potassium acetate (0.8 g) in dimethylformamide (7.5 ml) was added lead tetraacetate (2.17 g) in portions, followed by stirring for 1 hour at room temperature. The reaction mixture was diluted with water and extracted with ethyl acetate. The extract was washed with water, dried over anhydrous sodium sulfate and evaporated to give an oily residue which was purified by silica gel chromatography to give 1-(di-*p*-anisylmethyl)-3-ethenyl-4-acetoxy-2-azetidinone.

$IR_{max}^{CHCl_3}$ ($cm^{-1}$): 1760, 1608, 1298, 1240, 1174,, 1024, 974, 923
NMRδ ($CDCl_3$): 1.90(3H, s), 3.79(6H, s), 5.74(1H, br, s), 5.91(1H, s)

Example 4

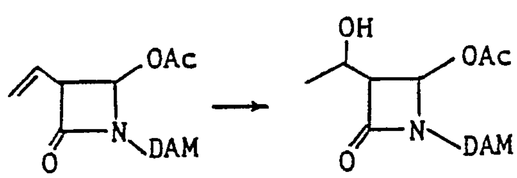

A solution of 1-(di-*p*-anisylmethyl)-3-ethenyl-4-acetoxy-2-azetidinone (3.80 g) and mercuric acetate (3.20 g) in tetrahydrofuran (10 ml) and water (4 ml) was stirred for 1 hour at room temperature. The reaction mixture was treated with 1N sodium hydroxide (9 ml) at 0°C and then a solution of sodium borohydride (0.4 g) in 1N aqueous sodium hydroxide. After stirring at the same temperature for 5 to 6 minutes, the reaction mixture was neutralized with dilute hydrochloric acid, diluted with diethyl ether and filtered, to remove any insoluble materials, upon the filter aid Celite. The filtrate was extracted with diethyl ether and the ether extract was washed successively with aqueous sodium bicarbonate and water, dried over anhydrous sodium sulfate and evaporated to give an oily residue which was then purified by silica gel chromatography to give 1-(di-*p*-anisylmethyl)-3-(1-hydroxyethyl)-4-acetoxy-2-azetidinone.

$IR_{max}^{CHCl_3}$ ($cm^{-1}$): 1752, 1608, 1357, 1302, 1242, 1174, 1028, 953
NMRδ($CDCl_3$): 1.25(3H, d, J=7), 1.90(3H, s), 3.07(1H, br, d, J=6.5), 3.78(6H, s), 5.83(1H, d), 5.88(1H, br, s)

Example 5

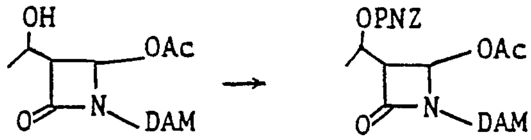

*p*-Nitrobenzyl chloroformate (3.52 g) in dichloromethane (9 ml) was added dropwise to a mixture of 1-(di-*p*-ansylmethyl)-3-(1-hydroxyethyl)-4-acetoxy-2-azetidinone (5.3 g) and dimethylaminopyridine (1.98 g) in dichloromethane (18 ml). The reaction mixture was stirred overnight at room temperature, washed successively with water, dilute hydrochloric acid and again water, dried over anhydrous sodium sulfate and evaporated to give an oily residue which was then purified by silica gel chromatography to give 1-(di-*p*-anisylmethyl)-3-(1-*p*-nitrobenzyloxycarbonyloxyethyl)-4-acetoxy-2-azetidinone.

$IR^{film}_{max}$ ($cm^{-1}$): 1770 (shoulder), 1740, 1610, 1563, 1020, 850, 818, 735
NMRδ($CDCl_3$): 1.42(3H, d, J=6), 1.85(3H, s), 3.28(1H, br, d, H=6), 5.22(2H, s), 5.87(1H, s), 6.11(1H, br, s)

Example 6

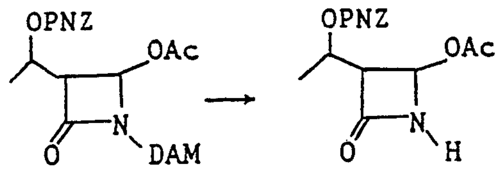

Ceric ammonium nitrate (12.1 g) was added to the solution of 1-(di-*p*-anisylmethyl)-3-(1-*p*-nitrobenzyloxycarbonyloxyethyl)-4-acetoxy-2-azetidinone (6.06 g) in 10% $H_2O$-acetonitrile (90 ml) in portions over 1 hour at room temperature, followed by stirring for 20 minutes. The reaction mixture was diluted with water and extracted with ethyl acetate. The extract was washed with water, dried over anhydrous sodium sulfate and evaporated to give an oily residue which was then purified by silica gel chromatography to give 3-(1-*p*-nitrobenzyloxycarbonyloxyethyl)-4-acetoxy-2-azetidinone.

$IR^{film}_{max}$ ($cm^{-1}$): 1774, 1745, 1602, 1513, 1344, 1258, 1029, 843
NMRδ($CDCl_3$): 1.45(3H, d, J=6), 2.09(3H, s), 3.37(1H, br, d, J=6, b), 5.87(1H, br, s), 6.96(1H, br, s)

Example 7

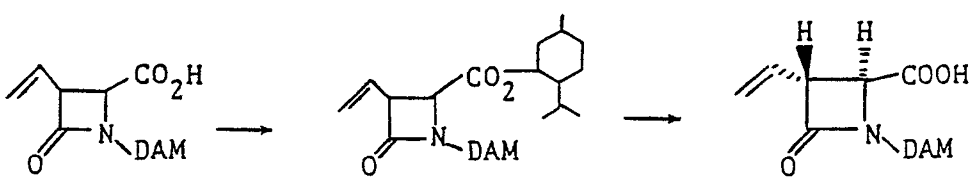

A solution of oxalyl chloride (4.25 g) in dichloromethane (5 ml) was added dropwise over 20 minutes to a mixture of 1-(di-*p*-anisylmethyl)-3-ethenyl-4-carboxyl-2-azetidinone (racemic form) (10.24 g) and a catalytic amount of dimethyl formamide in dichloromethane (45 ml) at room temperature. The resulting mixture was stirred for 1.5 hours at the same temperature and evaporated. The residue was dissolved in dichloromethane (30 ml) and cooled on an ice-bath. The mixture was added dropwise to a mixture of 4-dimethylaminopyridine (3.58 g) and *l*-(-)-menthol (4.59 g) in dichloromethane (30 ml), followed by stirring for 2 hours. The reaction mixture was washed successively with 2N hydrochloric acid and aqueous sodium bicarbonate, dried over anhydrous sodium sulfate and evaporated to give an oily residue which was then crystallized from methanol to give *l*-menthyl ether derivative (the ratio of the two isomers was 1:1) (m.p. 96~97°C).

The above crystals (10 g) were dissolved in methanol (400 ml) by heating and cooled at −5°C. Collection of the resulting crystals by filtration gave crystals of (3R, 4S)-1-(di-*p*-anisylmethyl)-3-ethenyl)-4-*l*-(-)-menthyloxycarbonyl-2-azetidinone, which was further purified by recrystallization from methanol to give pure (3R, 4S)-*l*-(-)-menthyl ester derivative [m.p. 114~115°C, specific rotation $[\alpha]^{22}_{D}$ = +20.2° (C=0.26, $CHCl_3$)].

The separation of the two isomers of *l*-(-)-menthyl ester derivative obtained as described above was also accomplished using high performance liquid chromatography (Column Lichrosorb® SI-60, solvent 1.5% isopropanol-hexane).

(3R, 4S)-1-(d-*p*-anisylmethyl)-3-ethenyl-4-carboxyl-2-azetidinone [specific rotation $[\alpha]^{22}_{D}$ = +63.3° (C=0.12, $CHCl_3$)] was obtained from (3R, 4S)-1-(di-*p*-anisylmethyl-3-ethenyl-4-menthyloxycarbonyl-2-azetidinone by a similar procedure to that described in Example 63.

Example 8

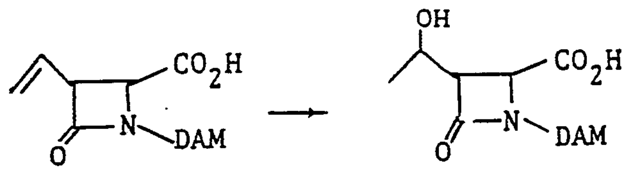

A mixture of 1(di-*p*-anisylmethyl)-3-ethenyl-4-carboxyl-2-azetidinone (1.0 g) and mercuric acetate (0.9 g) in tetrahydrofuran (8.8 ml) and water (2 ml) was refluxed for 8 hours. After addition of 1N aqueous sodium hydroxide (7.2 ml) at 0°C, a solution of sodium borohydride (0.1 g) in 1N aqueous sodium hydroxide (1 ml) was added to the mixture, followed by stirring at the same temperature for 5 to 6 minutes. The reaction mixture was neutralized with 6N hydrochloric acid, diluted with diethyl ether, filtered over Celite. The filtrate was extracted with diethyl ether and the extract was washed with saturated aqueous sodium chloride, dried over anhydrous sodium sulfate and evaporated to give an oily residue which was then purified by silica gel chromatography to give 1-(di-*p*-anisylmethyl)-3-(1-hydroxyethyl)-4-carboxyl-2-azetidinone (0.85 g).

$IR_{max}^{Nujol}$ ($cm^{-1}$): 3250, 1750, 1723, 1515, 1305, 1250, 1177, 1030, 835

NMRδ($CDCl_3$): 1.22 (3H, d, J=6Hz), 3.18(1H, m), 3.72(6H, s), 4.10(1H, d, J=2Hz), 5.75(1H, s)

Optically active (3S, 4S)-1-(di-*p*-anisylmethyl)-3-((R)-1-hydroxyethyl)-4-carboxyl-2-azetidinone [specific rotation $[\alpha]_D^{22}$ = +22.0° (C=0.14, $CHCl_3$)] was obtained from (3R, 4S)-1-(di-*p*-anisylmethyl)-3-ethenyl-4-carboxyl-2-azetidinone by the similar procedure as described above.

Example 9

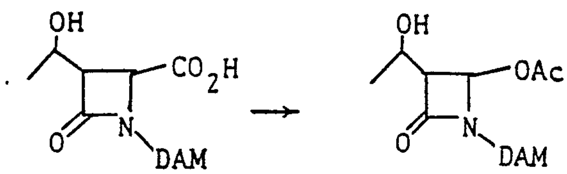

To a solution of 1-(di-*p*-anisylmethyl)-3-(1-hydroxyethyl)-4-carboxyl-2-azetidinone (4.0 g) in dimethylformamide (40 ml) was added potassium acetate (1.0 g). Then, lead tetraacetate (5.3 g) was added in portions to the mixture at 40°C, followed by stirring for 1 hour. The reaction mixture was treated with ethylene glycol to destroy the residual lead tetraacetate, diluted with ethyl acetate and saturated aqueous sodium chloride and filtered over Celite to remove insoluble material. The filtrate was extracted with ethyl acetate and the extract was washed with water, dried over anhydrous sodium sulfate and evaporated to give an oily residue which was then purified by silica gel chromatography to give 1-(di-*p*-anisylmethyl)-3-(1-hydroxyethyl)-4-acetoxy-2-azetidinone (3.03 g).

$IR_{max}^{CHCl_3}$ ($cm^{-1}$): 1752, 1357, 1302, 1242, 1174, 1028, 953

NMRδ($CDCl_3$): 1.26(3H, d, J=6.5Hz), 1.90(3H, s), 3.07(1H, br, d, J=6.5Hz), 3.78(6H, s), 4.07(1H, m), 5.83(1H, br, s), 5.88(1H, br, s)

Optically active (3R, 4S)-1-(di-*p*-anislymethyl)-3-((R)-1-hydroxyethyl)-4-acetoxy-2-azetidinone [specific rotation $[\alpha]_D^{22}$ = +26.0° (C=0.04, $CHCl_3$)] was obtained from (3R, 4S)-1-(di-*p*-anisylmethyl)-3-((R)-1-hydroxyethyl)-4-carboxy-2-azetidinone by the procedure similar to that described above. (3R, 4S)-3-((R)-1-*p*-nitrobenzyloxycarbonyloxyethyl)-4-acetoxy-2-azetidinone [specific rotation $[\alpha]_D^{22}$ = +36.6° (C=0.09, $CHCl_3$)] was obtained from the above optical active acetoxy derivative by the procedures similar to those described in Examples 66 and 67 via (3R, 4R)-1-(di-*p*-anisylmethyl)-3-((R)-1-*p*-nitrobenzyloxycarbonyloxyethyl)-4-acetoxy-2-azetidinone [specific rotation $[\alpha]_D^{22}$ = +40.5° (C=0.38, $CHCl_3$)].

Example 10

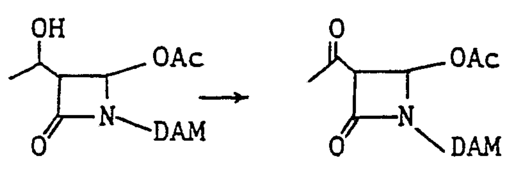

Jones' reagent (ca1 ml) was added dropwise at room temperature to a solution of 1-(di-*p*-anisylmethyl)-3-(1-hydroxyethyl)-4-acetoxy-2-azetidinone (0.40 g) in acetone (5 ml), followed by stirring for

10 minutes. The reaction mixture was diluted with water and extracted with ethyl acetate. The extract was washed with water, dried over anhydrous sodium sulfate and evaporated to give an oily residue which was then purified by silica gel chromatography to give 1-(di-*p*-anisylmethyl)-3-acetyl-4-acetoxy-2-azetidinone.

$IR_{max}^{film}$ ($cm^{-1}$): 1760, 1608, 1580, 1350, 1300, 1170, 1110, 1020, 815
NMRδ($CDCl_3$): 1.83(3H, s), 2.25(3H, s), 3.75(6H, s), 4.10(1H, d, J=1.5Hz), 5.78(1H, s), 6.12(1H, d, J=1.5 Hz)

Example 11

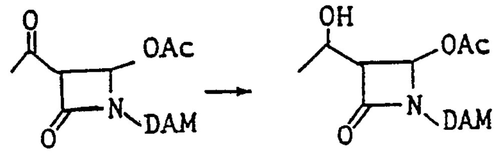

A mixture of sodium borohydride (38 mg) and 1-(di-*p*-anisylmethyl)-3-acetyl-4-acetoxy-2-azetidinone (397 mg) in isopropanol (6 ml) was stirred for 0.5 hour at room temperature. The reaction mixture was diluted with water and ethyl acetate. The extract was washed with water, dried over anhydrous sodium sulfate and evaporated to give 1-(di-*p*-anisylmethyl)-3-(1-hydroxyethyl)-4-acetoxy-2-azetidinone (the product was a mixture of two stereoisomers (1:1) of the hydroxyethyl group).

NMRδ($CDCl_3$): 3.10(1/2H, d, J=6.6Hz), 3.16(1/2H, d, J=4.4Hz)
Other peaks of NMR and IR spectra were similar to those of the compound obtained as described in Example 65.

Example 12

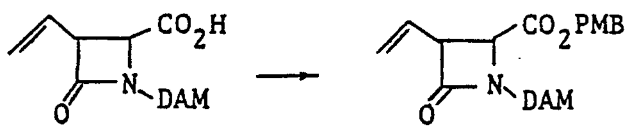

A mixture of 1-(di-*p*-anisylmethyl)-3-ethenyl-4-carboxyl-2-azetidinone (10 g), triethylamine (3.30 g) and *p*-methoxybenzyl chloride (5.19 g) in dimethylformamide (50 ml) was stirred at 70°C for 20 hours. The reaction mixture was diluted with ethyl acetate, washed successively with water, 2N hydrochloric acid and aqueous sodium bicarbonate, dried over anhydrous sodium sulfate and evaporated to give an oily residue which was then purified by silica gel chromatography to give 1-(di-*p*-anisylmethyl)-3-ethenyl-4-*p*-methoxybenzyloxycarbonyl-2-azetidinone.

$IR_{max}^{neat}$ ($cm^{-1}$): 1745, 1610, 1505, 1455, 1300, 1170, 1027, 822, 750
NMRδ($CDCl_3$): 3.72(3H, s), 3.75(6H, s), 4.83(2H, s), 5.1-6.0(3H, m), 5.78(1H, s)

Example 13

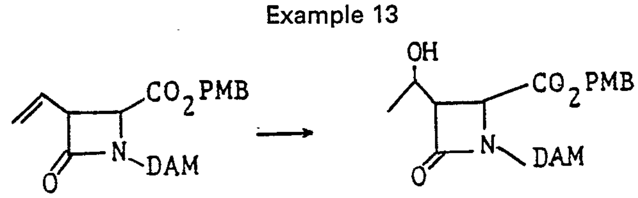

A mixture of 1-(di-*p*-anisylmethyl)-3-ethenyl-4-*p*-methoxybenzyloxycarbonyl-2-azetidinone (10 g) and mercuric acetate (6.6 g) in tetrahydrofuran (40 ml) and water (20 ml) was stirred at room temperature for 5 hours. The reaction mixture was treated with 1N aqueous sodium hydroxide (40 ml) and then sodium borohydride (0.78 g) in 1N aqueous sodium hydroxide (2 ml) at 0°C. The mixture was acidified with 2N hydrochloric acid, filtered to remove any insoluble materials. The filtrate was extracted with diethyl ether. The extract was washed successively with water and aqueous sodium bicarbonate, dried over anhydrous sodium sulfate and evaporated to give an oily residue which was then purified by silica gel chromatography to give 1 - (di - *p* - anisylmethyl) - 3 - (1 - hydroxyethyl) - 4 - *p* - methoxybenzyloxy-carbonyl - 2 - azetidinone.

$IR_{max}^{CHCl_3}$ ($cm^{-1}$): 3400, 1742, 1510, 1303, 1242, 1175, 1032, 824
NMR δ ($CDCl_3$): 1.17 (3H, d, J=6Hz), 3.13 (1H, dd, J=2.2 and 3.4Hz), 3.73 (3H, s), 3.84 (3H, s), 4.12 (1H, d, J=2.2Hz), 4.88 (2H, d), 5.82 (1H, s)

Example 14

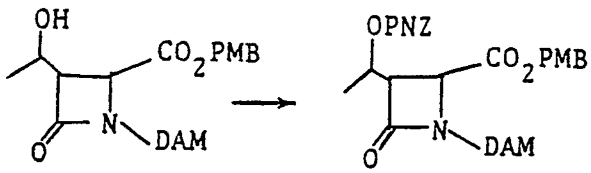

To a solution of 1-(di-*p*-anisylmethyl)-3-(1-hydroxyethyl)-4-*p*-methoxybenzyloxycarbonyl-2-azetidinone (4.5 g) in dichloromethane (25 ml) and 4-N,N-dimethylaminopyridine (1.31 g) was added dropwise *p*-nitrobenzylchloroformate (2.31 g) in dichloromethane (20 ml) with ice-cooling, followed by stirring for 2 hours. The reaction mixture was diluted with ethyl acetate, washed with 2N hydrochloric acid, dried over anhydrous sodium sulfate and evaporated to give an oily residue which was then purified by silica gel chromatography to give 1 - (di - *p* - anisylmethyl) - 3 - (1 - *p* - nitrobenzyloxycarbonyloxyethyl) - 4 - *p* - methoxybenzyloxycarbonyl - 2 - azetidinone.

$IR_{max}^{CHCl_3}$ ($cm^{-1}$): 1760, 1515, 1347, 1250, 1176, 1030, 847

NMR δ ($CDCl_3$): 1.38 (3H, d, J=7Hz), 3.32 (1H, dd, J=3 and 7Hz), 3.70 (3H, s), 3.73 (3H, s), 3.77 (3H, s), 4.10 (1H, d, J=3Hz), 4.87 (2H, s), 5.18 (2H, s), 5.78 (1H, s)

Example 15

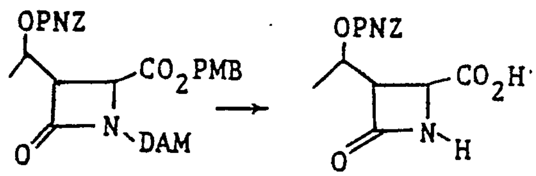

A mixture of 1-(di-*p*-anisylmethyl)-3-(1-*p*-nitrobenzyloxycarbonyloxyethyl)-4-*p*-methoxybenzyloxycarbonyl-2-azetidinone (0.64 g), anisol (0.43 g) and trifluoroacetic acid (1.2 ml) was stirred at 40°C for 20 hours. The reaction mixture was concentrated, diluted with ethylacetate, washed with water, dried over anhydrous sodium sulfate and evaporated to give an oily residue which was then purified by silica gel chromatography to give 3 - (1 - *p* - nitrobenzyloxycarbonyloxyethyl) - 4 - carboxyl - 2 - azetidinone.

$IR_{max}^{KBr}$ ($cm^{-1}$): 3380, 1760, 1520, 1350, 1270, 1016, 850

NMR δ ($(CD_3)_2SO$): 1.33 (3H, d, J=7Hz), 3.40 (1H, dd, J=2 and 5Hz), 3.95 (1H, d, J=2Hz), 5.28 (2H, s), 7.57 (2H, d, J=9Hz), 8.17 (2H, d, J=9Hz)

Example 16

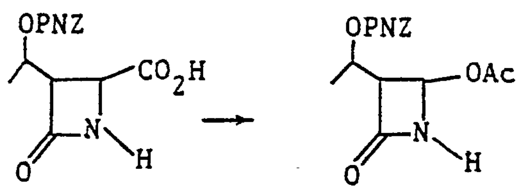

To a mixture of 3-(1-*p*-nitrobenzyloxycarbonyloxyethyl)-4-carboxyl-2-azetidinone (3.40 g) in dimethylformamide (20 ml) and potassium acetate (1.0 g) was added in portions lead tetraacetate (5.30 g) at 40°C. The reaction mixture was stirred for 1 hour, treated with ethylene glycol (ca 1 ml) for several minutes, diluted with ethyl acetate and saturated aqueous sodium chloride and filtered over Celite. The filtrate was extracted with ethyl acetate. The extract was washed with water, dried over anhydrous sodium sulfate and evaporated to give a residue which was then purified by silica gel chromatography to give 3 - (1 - *p* - nitrobenzyloxycarbonyloxyethyl) - 4 - acetoxy - 2 - azetidinone (2.46 g).

$IR_{max}^{CHCl_3}$ ($cm^{-1}$): 1752, 1352, 1302, 1242, 1174, 1028, 953

NMR δ ($CDCl_3$): 1.26 (3H, d, J=6.5Hz), 1.90 (3H, s), 3.07 (1H, br, d, J=6.5Hz), 3.78 (6H, s), 4.07 (1H, m), 5.83 (1H, br, s), 5.88 (1H, br, s)

## Claims

1. A process of preparing an azetidinone compound of the general formula:

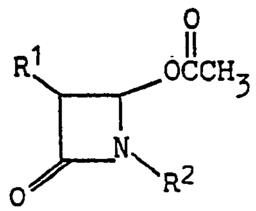

(a')

wherein $R^1$ is 1-hydroxyethyl, a protected 1-hydroxyethyl wherein the hydroxy group is protected by a conventional protecting group for a hydroxy group, or vinyl, and $R^2$ is hydrogen or mono- or diarylmethyl, which comprises oxidatively decarboxylating a carboxylic acid compound of the formula:

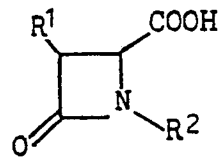

(b)

wherein $R^1$ and $R^2$ are as defined above, with lead tetraacetate in an inert solvent in the presence or absence of a base or a salt.

2. A process of preparing a compound of the formula:

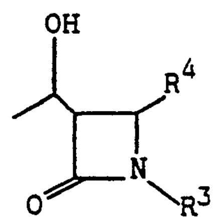

(b')

which can be used as starting material (b) in the process of Claim 1, wherein $R^3$ is mono- or diarylmethyl and $R^4$ is acetoxy, carboxyl or a protected carboxyl group, which comprises subjecting a compound of the formula:

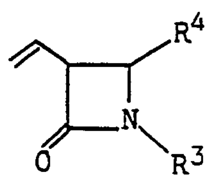

(c)

wherein $R^3$ and $R^4$ are as defined above, to oximercuration with mercuric acetate and then to reductive demercuration with a reducing agent in an inert solvent.

3. A process of preparing a compound of the general formula:

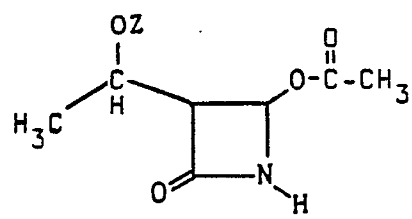

(a)

wherein Z is a conventional protecting group for a hydroxyl group, which comprises:

oxidizing a carboxylic acid compound of the formula:

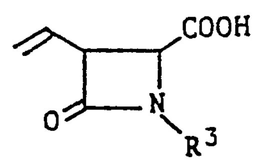

(d)

wherein and in formulae (e) and (f) $R^3$ is a mono- or diarylmethyl group, with lead tetraacetate in an inert solvent to produce a compound of the formula:

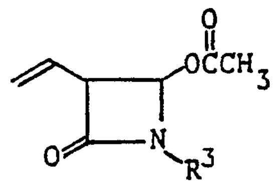

(e)

and then treating the compound of the formula (e) thus obtained with mercuric acetate, reducing the oxidized compound with a reducing agent in an inert solvent to produce a compound of the formula:

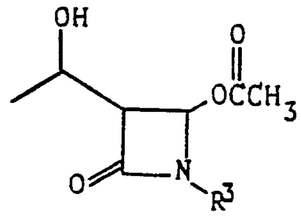

(f)

protecting the hydroxy group of the compound of the formula (f) thus obtained by a conventional hydroxy-protecting agent, and oxidizing the protected compound with ceric ammonium nitrate in an inert solvent.

4. A process of preparing a compound of the general formula (a') of Claim 1, which comprises:

treating a carboxylic acid compound of the formula (d) of Claim 3, with mercuric acetate and then reducing the oxidized compound with a reducing agent in an inert solvent to produce a compound of the formula:

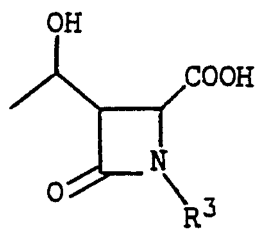

(g)

wherein and in formula (h) $R^3$ is as defined in Claim 3, oxidizing the compound of the formula (g) thus obtained with lead tetraacetate in an inert solvent to produce a compound of the formula:

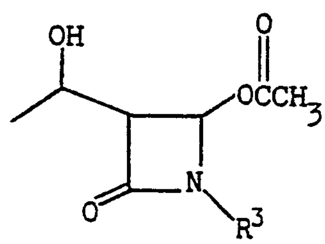

(h)

protecting the hydroxy group of the compound of the formula (h) thus obtained by a conventional hydroxy-protecting agent, and oxidizing the protected compound by ceric ammonium nitrate in an inert solvent.

5. A process of preparing a compound of the general formula (a') of Claim 1, which comprises:

treating a compound of the formula:

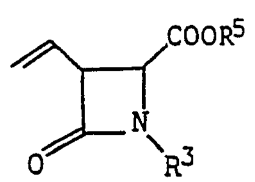

(d')

wherein and in formulae (j) and (k) $R^3$ is as defined in Claim 3 and $R^5$ is $C_1$—$C_4$ alkyl or aryl-($C_1$—$C_4$) alkyl, with mercuric acetate and then reducing the oxidized compound with a reducing agent in an inert solvent, protecting the hydroxy group of the resulting compound by a conventional hydroxy-protecting agent,

removing the protecting group for the carboxyl group ($R^5$) from the resulting hydroxy-protected compound to produce a compound of the formula:

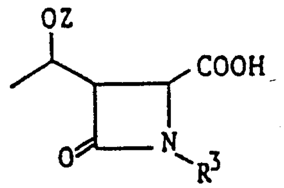

(j)

wherein Z is as defined in Claim 3, oxidizing the compound of the formula (j) thus obtained with ceric ammonium nitrate in an inert solvent to produce a compound of the formula:

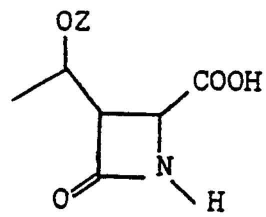

(k)

wherein Z is as defined above, and oxidizing the compound of the formula (k) thus obtained with lead tetraacetate.

6. A process as claimed in Claim 2, 3, 4 or 5, wherein the mercuric oxidation is carried out by reaction of the initial compound with 1 to 2 mols of mercuric acetate per mol of the azetidinone compound at a temperature of 0 to 100°C.

7. A process as claimed in Claim 6, wherein the reductive demercuration is carried out by reaction with 0.25 to 5 mols of sodium borohydride in the presence of an alkali metal hydroxide.

8. A process as carried out in any of Claims 1 or 3 to 7, wherein the oxidative decarboxylation is carried out with 1 to 3 mols of the lead tetraacetate per mol of the carboxylic azetidinone compound.

9. A process as claimed in any of the preceding claims, wherein the product of the general formula (a) is obtained in the *threo* steric form.

**Patentansprüche**

1. Verfahren zur Herstellung eines Azetidinons der allgemeinen Formel (a'),

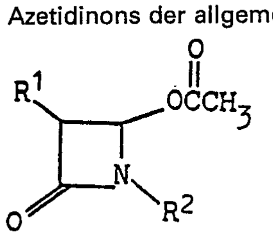

(a')

in der $R^1$ gegebenenfalls geschütztes 1-Hydroxyethyl, wobei die Hydroxylgruppe mit einer herkömmlichen Schutzgruppe für Hydroxylgruppen geschützt ist, oder Vinyl ist und $R^2$ Wasserstoff oder Mono- oder Diarylmethyl ist, gekennzeichnet durch oxidierende Decarboxylierung einer Carbonsäure der Formel (b),

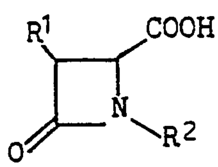

(b)

in der $R^1$ und $R^2$ wie oben definiert sind, mit Bleitetraazetat in einem inerten Lösungsmittel in Anwesenheit oder Abwesenheit einer Base oder eines Salzes.

2. Verfahren zur Herstellung einer Verbindung der Formel (b'), die als Ausgangsmaterial (b) im Verfahren nach Anspruch 1 verwendet werden kann,

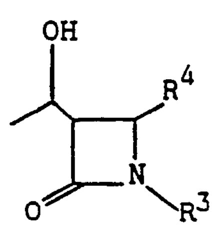

(b')

in der $R^3$ Mono- oder Diarylmethyl und $R^4$ Acetoxy oder gegebenenfalls geschütztes carboxyl ist, gekennzeichnet durch Umsetzung einer Verbindung der Formel (c),

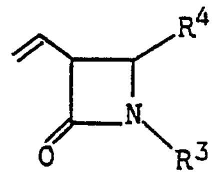

(c)

in der $R^3$ und $R^4$ wie oben definiert sind, mit Quecksilber(II)-azetat und Reduktion der erhaltenen Verbindung mit einem Reduktionsmittel in einem inerten Lösungsmittel.

3. Verfahren zur Herstellung einer Verbindung der allgemeinen Formel (a),

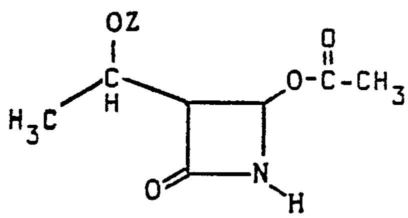

(a)

in der Z eine herkömmliche Schutzgruppe für eine Hydroxylgruppe ist, gekennzeichnet durch Oxidieren einer Carbonsäure der Formel (d),

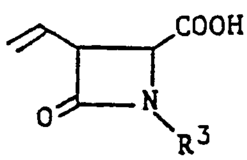

(d)

in der so wie in den Formeln (e) und (f) $R^3$ Mono- oder Diarylmethyl ist, mit Bleitetraazetat in einem inerten Lösungsmittel zu einer Verbindung der Formel (e),

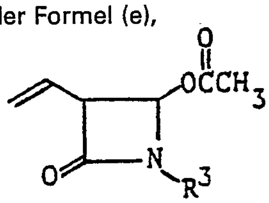

(e)

und Behandeln der so erhaltenen Verbindung der Formel (e) mit Quecksilber(II)-azetat, Reduzieren der oxidierten Verbindung mit einem Reduktionsmittel in einem inerten Lösungsmittel zu einer Verbindung der Formel (f),

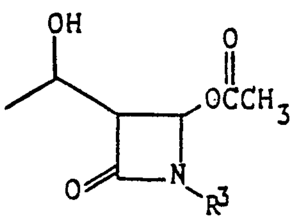

(f)

Schützen der Hydroxylgruppe der so erhaltenen Verbindung der Formel (f) mit einer herkömmlichen Schutzgruppe für Hydroxylgruppen und Oxidieren der geschützten Verbindung mit Cer(IV)-ammoniumnitrat in einem inerten Lösungsmittel.

4. Verfahren zur Herstellung einer Verbindung der allgemeinen Formel (a') nach Anspruch 1, gekennzeichnet durch

— Umsetzen einer Carbonsäure der Formel (d) nach Anspruch 3 mit Quecksilber(II)-azetat und Reduzieren der oxidierten Verbindung mit einem Reduktionsmittel in einem inerten Lösungsmittel zu einer Verbindung der Formel (g),

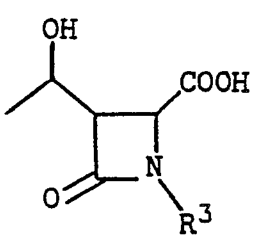

(g)

in der so wie in Formel (h) $R^3$ nach Anspruch 3 definiert ist,

— Oxidieren der so erhaltenen Verbindung der Formel (g) mit Bleitetraazetat in einem inerten Lösungsmittel zu einer Verbindung der Formel (h),

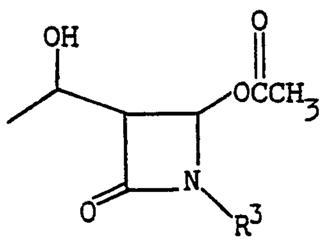

(h)

— Schützen der Hydroxylgruppe der so erhaltenen Verbindung der Formel (h) mit einer herkömmlichen Schutzgruppe für Hydroxylgruppen und

— Oxidieren der geschützten Verbindung mit Cer(IV)-ammoniumnitrat in einem inerten Lösungsmittel.

5. Verfahren zur Herstellung einer Verbindung der allgemeinen Formel (a') nach Anspruch 1, gekennzeichnet durch

— Umsetzen einer Verbindung der Formel (d')

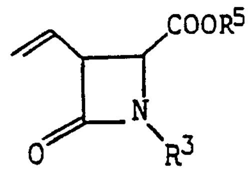

(d')

in der so wie in den Formeln (j) und (k) $R^3$ gemäß Anspruch 3 definiert ist und $R^5$ $C_{1-4}$-Alkyl oder Aryl-$C_{1-4}$-Alkyl ist, mit Quecksilber(II)-azetat,

— Reduzieren der oxidierten Verbindung mit einem Reduktionsmittel in einem inerten Lösungsmittel,

— Schützen der Hydroxylgruppe der entstandenen Verbindung mit einer herkömmlichen Schutzgruppe für Hydroxylgruppen,

— Entfernen der Schutzgruppe der Carboxylgruppe ($R^5$) aus der entstandenen hydroxylgeschützten Verbindung zu einer Verbindung der Formel (j),

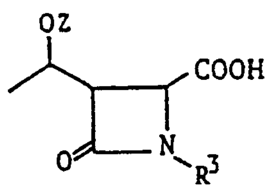

(j)

in der Z wie in Anspruch 3 definiert ist,

— Oxidieren der so erhaltenen Verbindung der Formel (j) mit Cer(IV)-ammoniumnitrat in einem inerten Lösungsmittel zu einer Verbindung der Formel (k),

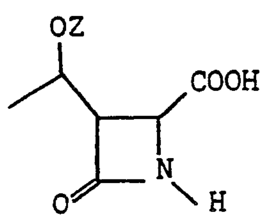

(k)

in der Z wie oben definiert ist, und

— Oxidieren der so erhaltenen Verbindung der Formel (k) mit Bleitetraazetat.

6. Verfahren nach Anspruch 2 bis 5, dadurch gekennzeichnet, daß die Oxidation mit dem Quecksilber(II)-Salz durch Umsetzung der Ausgangsverbindung mit 1 bis 2 Mol Quecksilber(II)-azetat je Mol Azetidinon bei einer Temperatur von 0 bis 100°C durchgeführt wird.

7. Verfahren nach Anspruch 6, dadurch gekennzeichnet, daß die Reduktion durch Umsetzung mit 0,25 bis 5 Mol Natriumborhydrid in Gegenwart eines Alkalimetallhydroxids durchgeführt wird.

8. Verfahren nach einem der Ansprüche 1 oder 3 bis 7, dadurch gekennzeichnet, daß die oxidierende Decarboxylierung mit 1 bis 3 Mol Bleitetraazetat je Mol Azetidinon-carbonsäure durchgeführt wird.

9. Verfahren nach einem der vorstehenden Ansprüche, dadurch gekennzeichnet, daß das Produkt der allgemeinen Formel (a) in der sterischen threo-Form erhalten wird.

**Revendications**

1. Procédé de préparation d'un composé azétidinone de formule générale:

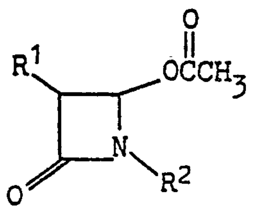

(a')

dans laquelle $R^1$ est un 1-hydroxyéthyle, un 1-hydroxyéthyle protégé où le groupe hydroxy est protégé par un groupe de protection conventionnel pour un groupe hydroxy, ou un vinyle, et $R^2$ est un hydrogène ou un mono- ou diarylméthyle, qui comprend la décarboxylation oxydative d'un composé acide carboxylique de formule:

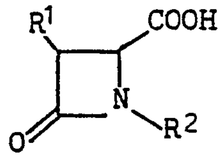

(b)

dans laquelle $R^1$ et $R^2$ sont comme définis ci-dessus, avec du tétraacétate de plomb dans un solvant inerte en présence ou en absence d'une base ou d'un sel.

2. Procédé de préparation d'un composé de formule:

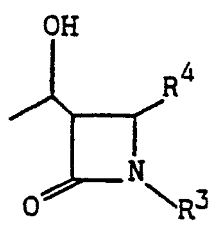

(b')

qui peut être utilisé comme matériau de départ (b) dans le procédé selon la revendication 1, dans laquelle $R^3$ est un mono- ou diarylméthyle et $R^4$ est un groupe acétoxy, carboxyle ou carboxyle protégé, dans lequel on soumet un composé de formule:

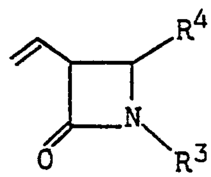

(c)

dans laquelle $R^3$ et $R^4$ sont comme définis ci-dessus, à une oxymercuration avec de l'acétate mercurique, puis à une démercuration réductive avec un agent de réduction dans un solvant inerte.

3. Procédé de préparation d'un composé de formule générale:

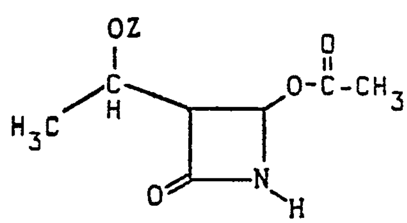

(a)

dans laquelle Z est un groupe de protection conventionnel pour un groupe hydroxyle, qui comprend l'oxydation d'un composé acide carboxylique de formule:

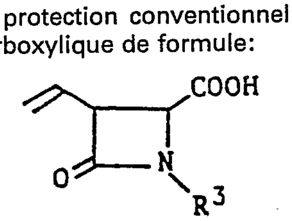

(d)

dans laquelle, de même que dans les formules (e) et (f), $R^3$ est un groupe mono- ou diarylméthyle, avec du tétraacétate de plomb dans un solvant inerte pour produire un composé de formule:

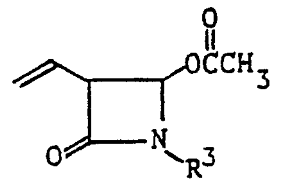

(e)

puis le traitement du composé de formule (e) ainsi obtenu avec de l'acétate mercurique, la réduction du composé oxydé avec un agent réducteur dans un solvant inerte pour produire un composé de formule:

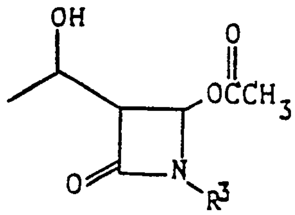

(f)

la protection du groupe hydroxy du composé de formule (f) ainsi obtenu par un agent de protection conventionnel du groupe hydroxy, et l'oxydation du composé protégé avec du nitrate d'ammonium cérique dans un solvant inerte.

4. Procédé de préparation d'un composé de formule générale (a') selon la revendication 1, qui comprend le traitement d'un composé acide carboxylique de formule (d) selon la revendication 3, avec de l'acétate mercurique, puis la réduction du composé oxydé avec un agent réducteur dans un solvant inerte pour produire un composé de formule:

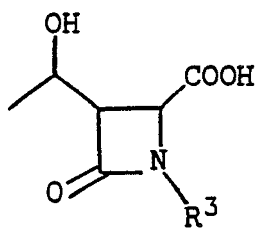

(g)

dans laquelle, de même que dans la formule (h), $R^3$ est comme défini dans la revendication 3, l'oxydation du composé de formule (g) ainsi obtenu avec du tétraacétate de plomb dans un solvant inerte pour produire un composé de formule:

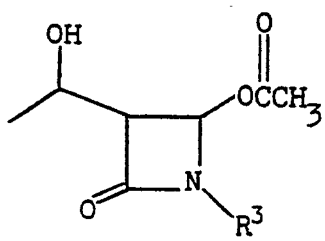

(h)

la protection du groupe hydroxy du composé de formule (h) ainsi obtenu par un agent de protection conventionnel du groupe hydroxy et l'oxydation du composé protégé par du nitrate d'ammonium cérique dans un solvant inerte.

5. Procédé de préparation d'un composé de formule générale (a') selon la revendication 1, qui comprend le traitement d'un composé de formule:

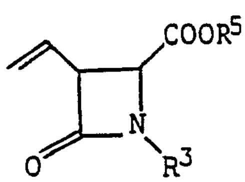

(d')

dans laquelle, ainsi que dans les formules (j) et (k), $R^3$ est comme défini dans la revendication 3 et $R^5$ est un alkyle en $C_1$—$C_4$ ou un aryl-alkyle ($C_1$—$C_4$), avec de l'acétate mercurique, puis la réduction du composé oxydé avec un agent réducteur dans un solvant inerte, la protection du groupe hydroxy du composé

résultant par un agent de protection conventionnel du groupe hydroxy, l'élimination du groupe de protection pour le groupe carboxyle ($R^5$) du composé obtenu avec un groupe hydroxy protégé pour produire un composé de formule:

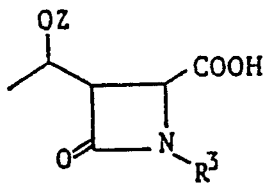

(j)

dans laquelle Z est comme défini dans la revendication 3, l'oxydation du composé de formule (j) ainsi obtenu avec du nitrate d'ammonium cérique dans un solvant inerte pour produire un composé de formule:

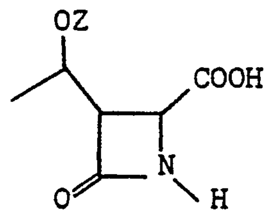

(k)

dans laquelle Z est comme défini ci-dessus, et l'oxydation du composé de formule (k) ainsi obtenu avec du tétraacétate de plomb.

6. Procédé selon la revendication 2, 3, 4, ou 5, dans lequel l'oxydation mercurique est effectuée par réaction du composé initial avec 1 à 2 moles d'acétate mercurique per mole de composé azétidinone à une température de 0 à 100°C.

7. Procédé selon la revendication 6, dans lequel la démercuration réductive est effectuée par réaction avec 0,25 à 5 moles de borohydrure de sodium en présence d'un hydroxyde de métal alcalin.

8. Procédé selon l'une des revendications 1 ou 3 à 7, dans lequel la décarboxylation oxydative est effectuée avec 1 à 3 moles de tétraacétate de plomb par mole de composé azétidinone carboxylique.

9. Procédé selon l'une des revendications précédentes, dans lequel le produit de formule générale (a) est obtenu sous la forme stérique *thréo*.